# EUROPEAN PATENT APPLICATION

(11) **EP 3 905 342 A1**
(43) Date of publication of application: **03.11.2021**
(21) Application number: 19903424.0
(22) Date of filing: 24.12.2019
(51) Int. Cl.: H01L 31/12, A61B 5/026

(54) **OPTICAL SENSOR DEVICE**

(30) Priority: 25.12.2018 JP 2018241588; 27.12.2018 JP 2018244187
(71) Applicant: Kyocera Corporation, Kyoto-shi, Kyoto 612-8501 (JP)
(72) Inventor: TODA, Keisuke, Kyoto-shi, Kyoto 612-8501 (JP)
(74) Representative: Viering, Jentschura & Partner mbB Patent- und Rechtsanwälte
(86) International application number: PCT/JP2019/050612
(87) International publication number: WO 2020/138086

(57) **Abstract**

An optical sensor device (10) includes an optical sensor (1) and a mounting board (7). The optical sensor (1) includes a substrate (2), a light receiver (6), a light emitter (5), and a transparent substrate (3). The substrate (2) has a first recess (21) and a second recess (22) at a distance from the first recess (21). The light receiver (6) is located in the first recess (21). The light emitter (5) is located in the second recess (22) and at a distance from the light receiver (6). The transparent substrate (3) is located on a first upper surface (23) of the substrate (2), bonded to the substrate (2), and covers the first recess (21) and the second recess (22). The optical sensor (1) is mounted with a first lower surface (11) being oblique to a second lower surface (72) of the mounting board (7).

## Description

### FIELD

The present disclosure relates to an optical sensor device.

### BACKGROUND

Optical sensor devices such as measurement sensors that easily and speedily measure biometric information including blood flow have been awaited. Measurement of blood flow uses, for example, the Doppler effect of light. When blood is illuminated with light, the light is scattered by blood cells, such as red blood cells. The frequency of the illuminating light and the frequency of the scattered light are used to calculate the traveling speed of the blood cells. An optical sensor for measuring blood flow or other items described in, for example, Japanese Unexamined Patent Application Publication No. 2011-134463 includes a substrate accommodating a light receiver and a light emitter, and a transparent substrate bonded to the upper surface of the substrate. An optical sensor device includes such optical sensors mounted on a mounting board.

However, the above optical sensor device includes the mounting board and the optical sensor arranged parallel to each other and bonded with a bond or another interconnection. In this structure, the light receiver may receive much noise light reflected on, for example, a surface skin and may cause the intensity of signal light scattered by blood cells to be relatively small.

### BRIEF SUMMARY

An optical sensor device according to an aspect of the present disclosure includes an optical sensor and a mounting board receiving the optical sensor. The optical sensor includes a first upper surface and a first lower surface, a substrate, a light receiver, a light emitter, and a transparent substrate. The mounting board has a second upper surface and a second lower surface. The substrate has a first recess and a second recess at a distance from the first recess. The light receiver is located in the first recess. The light emitter is located in the second recess and at a distance from the light receiver. The transparent substrate is located on the first upper surface of the substrate, bonded to the substrate, and covers the first recess and the second recess. The optical sensor is mounted with the first lower surface being oblique to the second lower surface of the mounting board.

### BRIEF DESCRIPTION OF DRAWINGS

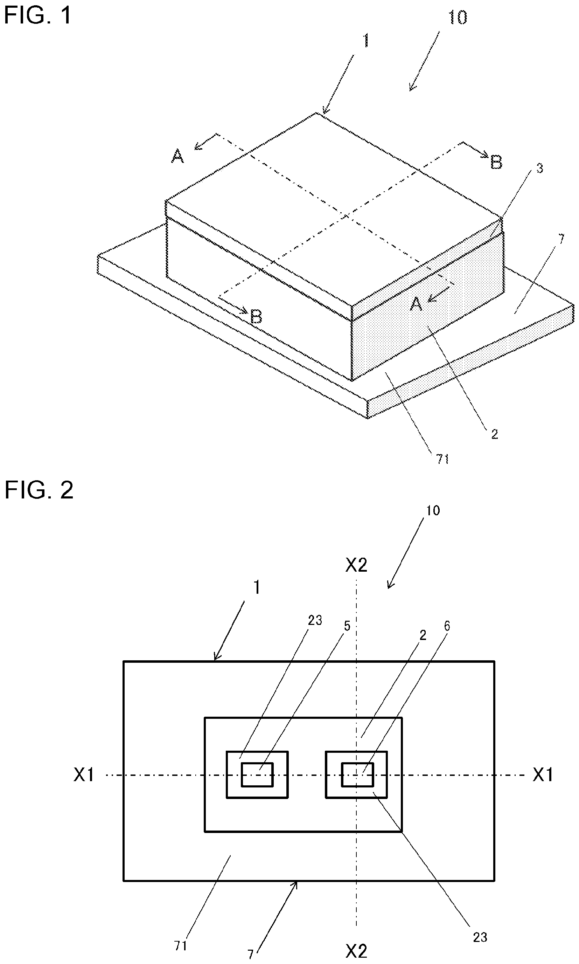

FIG. 1 is a perspective view of an optical sensor device according to an embodiment of the present disclosure.
FIG. 2 is a top view of the optical sensor device according to the embodiment of the present disclosure.
FIG. 3 is a cross-sectional view of the optical sensor device according to the embodiment of the present disclosure, taken along line A-A in FIG. 1.
FIG. 4 is a cross-sectional view of an optical sensor device according to an embodiment of present disclosure, taken along line A-A in FIG. 1.

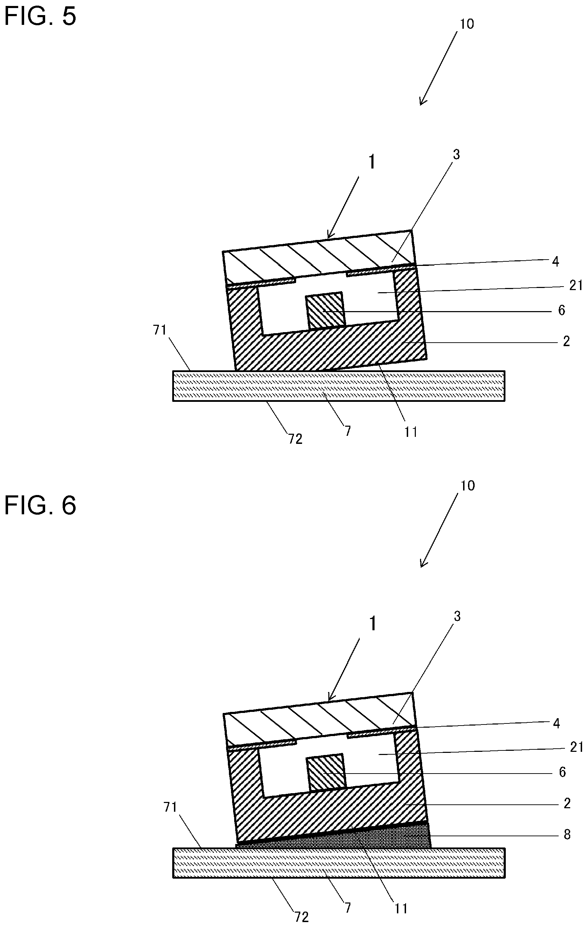

FIG. 5 is a cross-sectional view of an optical sensor device according to an embodiment of the present disclosure, taken along line B-B in FIG. 1.
FIG. 6 is a cross-sectional view of an optical sensor device according to an embodiment of the present disclosure, taken along line B-B in FIG. 1.
FIG. 7 is a cross-sectional view of an optical sensor device according to an embodiment of the present disclosure, taken along line B-B in FIG. 1.
FIG. 8 is a cross-sectional view of an optical sensor device according to another embodiment of the present disclosure.
FIG. 9 is a perspective view of an optical sensor device according to an embodiment of the present disclosure.
FIG. 10 is a cross-sectional view of the optical sensor device according to the embodiment of the present disclosure, taken along line C-C in FIG. 9.
FIG. 11 is a cross-sectional view of an optical sensor device according to another embodiment of the present disclosure, taken along line C-C in FIG. 9.
FIG. 12 is a cross-sectional view of an optical sensor device according to another embodiment of the present disclosure, taken along line C-C in FIG. 9.
FIG. 13 is a cross-sectional view of an optical sensor device according to another embodiment of the present disclosure, taken along line D-D in FIG. 9.
FIG. 14 is a cross-sectional view of an optical sensor device according to another embodiment of the present disclosure, taken along line D-D in FIG. 9.
FIG. 15 is a cross-sectional view of an optical sensor device according to another embodiment of the present disclosure, taken along line D-D in FIG. 9.
FIG. 16 is a cross-sectional view of an optical sensor device according to another embodiment of the present disclosure, taken along line D-D in FIG. 9.
FIG. 17 is a cross-sectional view of an optical sensor device according to another embodiment of the present disclosure, taken along line D-D in FIG. 9.

### DETAILED DESCRIPTION

An optical sensor device 10 according to an embodiment of the present disclosure includes an optical sensor 1 and a mounting board 7 receiving the optical sensor 1. The optical sensor 1 includes a substrate 2, a transparent substrate 3, a light emitter 5, and a light receiver 6. The optical sensor 1 has a first upper surface 23 and a first lower surface 11. The first upper surface 23 may be the upper surface of the substrate 2, and the first lower surface 11 may be the lower surface of the substrate 2.

The substrate 2 may be a rectangular plate in a plan view, and may include multiple dielectric layers stacked on one another. The substrate 2 has, for example, a dimension of 0.5 to 5 mm in a plan view, and a thickness of 0.5 to 5 mm. The substrate 2 may be, for example, a wiring board including ceramic dielectric layers, or an organic wiring board including dielectric layers of an insulating resin.

The substrate 2 serving as a wiring board of a ceramic material (ceramic wiring board) includes ceramic dielectric layers with conductors, such as connection pads, internal interconnections, and signal interconnections. The ceramic wiring board includes multiple ceramic dielectric layers.

Examples of the ceramic material used for the ceramic wiring board include sintered aluminum oxide, sintered mullite, sintered silicon carbide, sintered aluminum nitride, sintered silicon nitride, and sintered glass ceramic.

The substrate 2 serving as a wiring board of an organic material (organic wiring board) includes organic insulating layers with interconnection conductors, such as signal interconnections (described later). The organic wiring board is formed from multiple organic dielectric layers. The organic wiring board may be any wiring board having dielectric layers formed from an organic material, such as a printed wiring board, a build-up wiring board, or a flexible wiring board. Examples of the organic material used for an organic wiring board include an epoxy resin, a polyimide resin, a polyester resin, an acryl resin, a phenol resin, and a fluorine-based resin.

The substrate 2 has at least two recesses, or specifically, a first recess 21 for accommodating the light receiver 6 and a second recess 22 for accommodating the light emitter 5. The first recess 21 and the second recess 22 are open in the same main surface of the substrate 2 (the first upper surface 23 of the optical sensor 1).

The optical sensor device 10 according to the embodiment of the present disclosure is used as a measurement sensor to measure fluid flow, such as blood flow, using the Doppler effect of light. To use the Doppler effect of light, the measurement sensor includes a light emitter, which illuminates an object to be measured with light, and a light receiver, which receives light scattered by the object. When measuring, in particular, blood flow, the measurement sensor illuminates a body part, such as a finger, with external light, and receives light scattered by blood cells in the blood flowing through blood vessels under the skin to measure the blood flow based on changes in the frequency. In the optical sensor device 10, the light emitter 5 and the light receiver 6 are at a predetermined distance from each other based on the positional relationship between the illumination light and the scattered light. The first recess 21 and the second recess 22 are positioned in accordance with the positional relationship between the light receiver 6 and the light emitter 5.

The first recess 21 and the second recess 22 may be sized appropriately in accordance with the size of the light receiver 6 and the size of the light emitter 5 to be accommodated in the recesses. When, for example, a vertical-cavity surface-emitting laser (VCSEL) element is used as the light emitter 5, the first recess 21 may have a rectangular opening or a square opening, which has, for example, a longitudinal dimension of 0.3 to 2.0 mm, a lateral dimension of 0.3 to 2.0 mm, and a depth of 0.3 to 1.0 mm. When a light-emitting diode (LED) or a surface incident photodiode is used as the light receiver 6, the second recess 22 may have a rectangular opening or a square opening, which has, for example, a longitudinal dimension of 0.3 to 2.0 mm, a lateral dimension of 0.3 to 2.0 mm, and a depth of 0.4 to 1.5 mm. The first recess 21 and the second recess 22 (the light receiver 6 and the light emitter 5) may be at any distance long enough to prevent light from the light emitter 5 from directly entering the light receiver 6. A light-shield wall may be placed between the first recess 21 and the second recess 22 (between the light receiver 6 and the light emitter 5) to allow for a shorter distance between the first recess 21 and the second recess 22 (between the light receiver 6 and the light emitter 5).

The first recess 21 and the second recess 22 may each have, for example, a circular, square, or rectangular opening, or an opening having another shape. The first recess 21 and the second recess 22 may each have a uniform cross section parallel to the main surface of the substrate 2 in the depth direction. The first recess 21 and the second recess 22 may each have a step, or in other words, each have the same cross section as the opening to a predetermined depth and then having a smaller, uniform cross section from the predetermined depth to the bottom. In one embodiment of the present disclosure, a recess with a step as the first recess 21 has a mount for the light receiver 6 at the bottom. A recess with a step as the second recess 22 has a mount for the light emitter 5 at the bottom. Each recess receives a connection pad on the step for electrical connection to the light emitter 5 or the light receiver 6.

The substrate 2 may include signal interconnections electrically connected to the light emitter 5 or the light receiver 6 to transmit electric signals input into the light emitter 5 or output from the light receiver 6. Each signal interconnection may include a bonding wire, which is a connector connected to the light emitter 5 or the light receiver 6, a connection pad, to which the bonding wire is connected, a via conductor, which is electrically connected to the connection pad and extends from immediately below the connection pad to the lower surface of the substrate 2, and an external connection terminal, which is electrically connected to the via conductor. Each external connection terminal is located on the lower surface of the substrate 2 and electrically connected, with a terminal bond such as solder, to a connection terminal on an external mounting board, on which a measurement sensor including the optical sensor device 10 is mountable.

To improve the wettability of a bond such as solder and to improve the corrosion resistance, the external connection terminal may include a nickel layer having a thickness of 0.5 to 10 µm and a gold layer having a thickness of 0.5 to 5 µm, which may be deposited in sequence by plating.

The transparent substrate 3 covers the upper surface of the substrate 2 (the first upper surface 23 of the optical sensor 1) and is bonded to the first upper surface 23 with a bond. The transparent substrate 3 covers and seals the first recess 21 and the second recess 22 accommodating the light receiver 6 and the light emitter 5. The transparent substrate 3 may be a plate of an insulating material. The transparent substrate 3 may be formed from a light-transmissive material that transmits light emitted from the light emitter 5 accommodated in the second recess 22, and light to be received by the light receiver 6 accommodated in the first recess 21.

The light emitter 5 may be a semiconductor laser element such as a VCSEL. The light receiver 6 may be a photodiode such as a silicon photodiode, a GaAs photodiode, an InGaAs photodiode, or a germanium photodiode. The light emitter 5 and the light receiver 6 may be selected as appropriate in accordance with the type of an object to be measured or the parameters to be measured.

For example, the VCSEL that can emit a laser beam with a wavelength of 850 nm may be used as the light emitter 5 for measuring blood flow using the Doppler effect of light. To measure another object, another device that emits a laser beam with a wavelength appropriate for the measurement object may be selected as the light emitter 5. With a laser beam emitted from the light emitter 5 and having its wavelength unchanged, any light receiver that can receive such a beam may be used as the light receiver 6. With a laser beam emitted from the light emitter 5 and having its wavelength changed, any light receiver that can receive such a beam with its wavelength changed may be used as the light receiver 6.

Although the light emitter 5 and the light receiver 6 are electrically connected to the connection pad with, for example, bonding wires in the present embodiment, the connection may be achieved with another method, such as flip chip connection, bump connection, or connection using an anisotropic conductive film.

The transparent substrate 3 is to transmit the illumination light and the scattered light to and from a measurement object. The characteristics of the illumination light and the scattered light depend on the light emitter mounted. The transparent substrate 3 may thus at least transmit the light emitted from the light emitter mounted. The transparent substrate 3 may be formed from an insulating material having a light transmissivity of at least 70%, or more specifically at least 90% for the wavelength of light emitted from the light emitter.

Examples of the insulating material for the transparent substrate 3 include a transparent ceramic material such as sapphire, a glass material, and a resin material. Examples of the glass material include borosilicate glass, crystallized glass, quartz, and soda glass. Examples of the resin material include a polycarbonate resin, an unsaturated polyester resin, and an epoxy resin. The transparent substrate 3 is, for example, rectangular in a plan view and has dimensions of 0.5 × 1 mm to 5 × 5 mm. The transparent substrate 3 has a thickness of 0.2 to 5 mm.

An adhesive is used to bond the substrate 2 and the transparent substrate 3 together. More specifically, the adhesive is used to bond the first upper surface 23 and the lower surface of the transparent substrate 3 at their outer peripheries. The adhesive is continuously applied along the first upper surface 23 and serves as a sealant that provides airtightness and water tightness inside the first recess 21 and the second recess 22 in the substrate 2. The light receiver 6 and the light emitter 5 to be accommodated in the first recess 21 and the second recess 22 are susceptible to moisture. To prevent entry of external moisture, the adhesive is continuously applied.

The adhesive may also be light-shielding. This light-shielding adhesive reduces entry of external light in the first recess 21 or the second recess 22 through the gap between the substrate 2 and the transparent substrate 3.

The adhesive may absorb light for light-shielding. The adhesive may reflect light for light-shielding to prevent entry of external light. However, the adhesive in this case may reflect any stray light inside the measurement sensor, which may then be received by the light receiver. The adhesive that absorbs light prevents entry of external light and also absorbs internally occurring stray light.

The adhesive may be formed from materials containing a material that absorbs light for light-shielding. The adhesive may be a resin adhesive, such as an epoxy resin or a conductive silicone resin, which bonds the substrate 2 and the transparent substrate 3 together, with a light-absorbing material dispersed in the resin adhesive. Examples of the light-absorbing material include inorganic pigments. Examples of the inorganic pigments include carbon pigments such as carbon black, nitride pigments such as titanium black, and metal oxide pigments such as Cr-Fe-Co, Cu-Co-Mn, Fe-Co-Mn, and Fe-Co-Ni-Cr pigments. A conductive bond material may be formed from a metal material such as solder. Examples of such conductive bond materials include a brazing material, such as Sn-Ag, Sn-Ag-Cu, Au-Sn, Au-Sn-Ag, or Au-Si.

The transparent substrate 3 includes a light shield 4 on its lower surface. The light shield 4 may be formed by, for example, vapor deposition, sputtering, or baking of a metal material such as Cr, Ti, Al, Cu, Co, Ag, Au, Pd, Pt, Ru, Sn, Ta, Fe, In, Ni, and W or an alloy of these metals. The light shield 4 has a thickness of, for example, 50 to 1,000 nm. The light shield 4 may align with the light emitter 5. The light shield 4 aligning with the light emitter 5 refers to the light shield 4 covering a part of the light emitter 5. The light shield 4 has through-holes that allow at least light from the light emitter 5 and reflected light reaching the light receiver 6 to pass through.

The light shield 4 may cover an area except an area through which light from the light emitter 5 is to be transmitted. This can reduce light leaking through the covered area of the transparent substrate 3, thus reducing leaking light unintendedly reflected on a measurement object and entering the light receiver 6.

The optical sensor 1 is mounted on the mounting board 7. The mounting board 7 is, for example, rectangular in a plan view, with the dimensions of 3 × 7 mm to 50 × 70 mm and a thickness of 0.3 to 2 mm. The mounting board may be formed from a material containing an organic material or a ceramic material.

The mounting board 7 and the optical sensor 1 are bonded together with, for example, a bond 8. The bond 8 may be formed from an epoxy resin or a silicone resin, or from a metal material such as solder. The bond 8 may also include a brazing material, such as Sn-Ag, Sn-Ag-Cu, Au-Sn, Au-Sn-Ag, or Au-Si.

The optical sensor 1 is mounted on the mounting board 7 with its first lower surface 11 being oblique to at least one of a second upper surface 71 or a second lower surface 72 of the mounting board 7 in a cross-sectional view taken along a second imaginary straight line X2 perpendicular to a first imaginary straight line X1, which connects the center of the light receiver 6 and the center of the light emitter 5 in a plan view. The optical sensor 1 thus has the first lower surface 11 oblique to at least the second lower surface 72 of the mounting board 7, or in other words, the optical sensor 1 has its optical axis oblique to the surface of the object (fluid). The light emitter 5 is aligned with the surface of the object in a transparent plan view, and is oblique to the surface of the object in a cross-sectional view. The optical sensor 1 may have the first lower surface 11 partly separated from the mounting board 7 or may have the bond 8 with different thicknesses including thinner portions and thicker portions to cause the optical sensor 1 to have the first lower surface 11 oblique to the second lower surface 72 of the mounting board 7. The bond 8 may include multiple solder balls with different diameters to cause the optical sensor 1 to be inclined. A solder ball refers to solder shaped in a ball. In other words, a solder ball is a spherical solder member serving as a bond. More specifically, the multiple solder balls include first solder balls at a first position on the first lower surface 11 at which the optical sensor 1 is at a first distance from the mounting board 7 and second solder balls at a second position on the first lower surface 11 at which the optical sensor 1 is at a second distance greater than the first distance from the mounting board 7. The second solder balls each have a larger diameter than the first solder balls. The multiple solder balls may include a solder ball with a larger diameter at a position on the first lower surface 11 at which the optical sensor 1 is at a greater distance from the mounting board 7. The bond 8 may be located across the first lower surface 11 of the optical sensor 1.

In the optical sensor device 10 according to the embodiment of the present disclosure, the optical sensor 1 has the first lower surface 11 oblique to the second lower surface 72 of the mounting board 7 as described above. Light from the light emitter 5 thus has its optical axis oblique to the surface of the object (fluid or flow passage), allowing the light component specularly reflected on the surface to be redirected outside the optical sensor 1. In other words, this structure has a higher ratio of detectable signal light than the structure redirecting the specularly reflected light component onto the light receiver 6, and improves the detection of the intensity of the signal light.

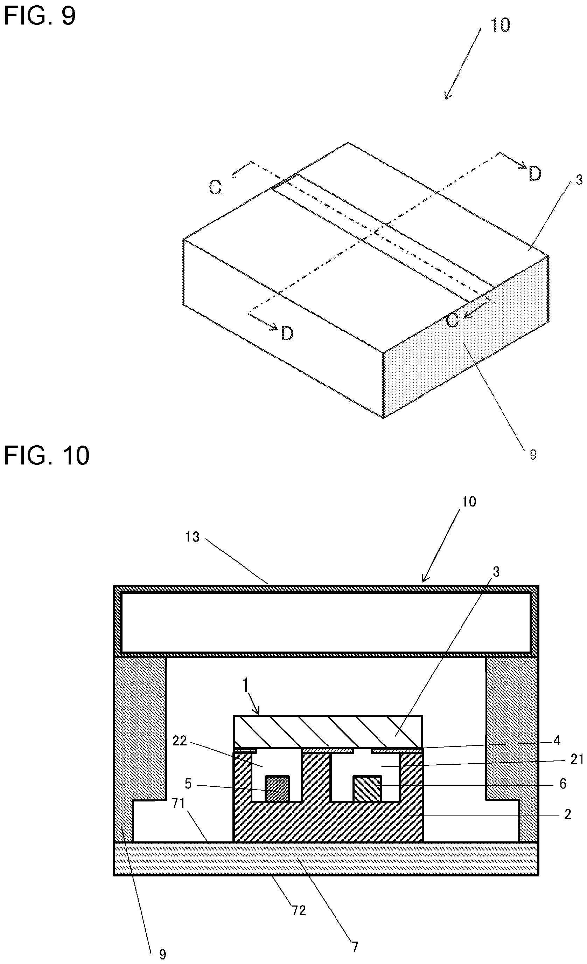

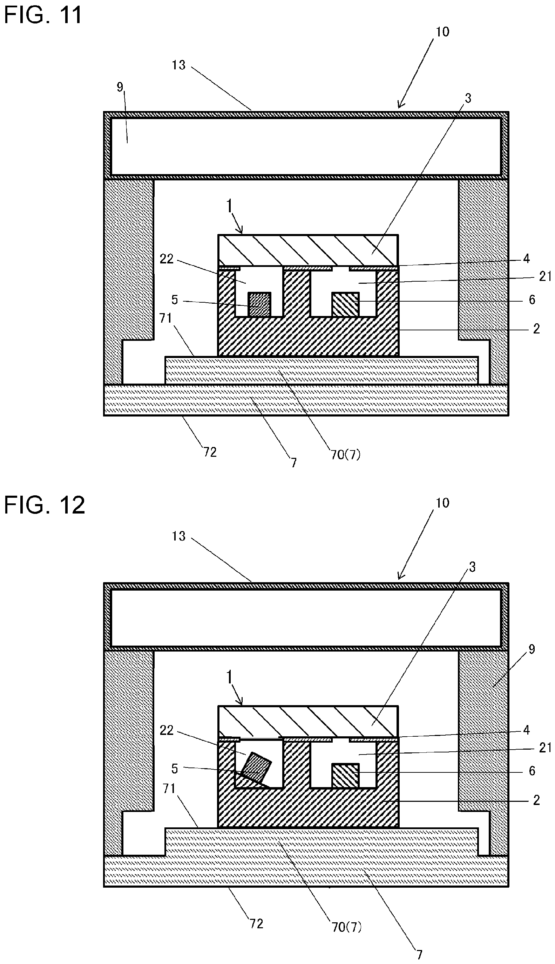

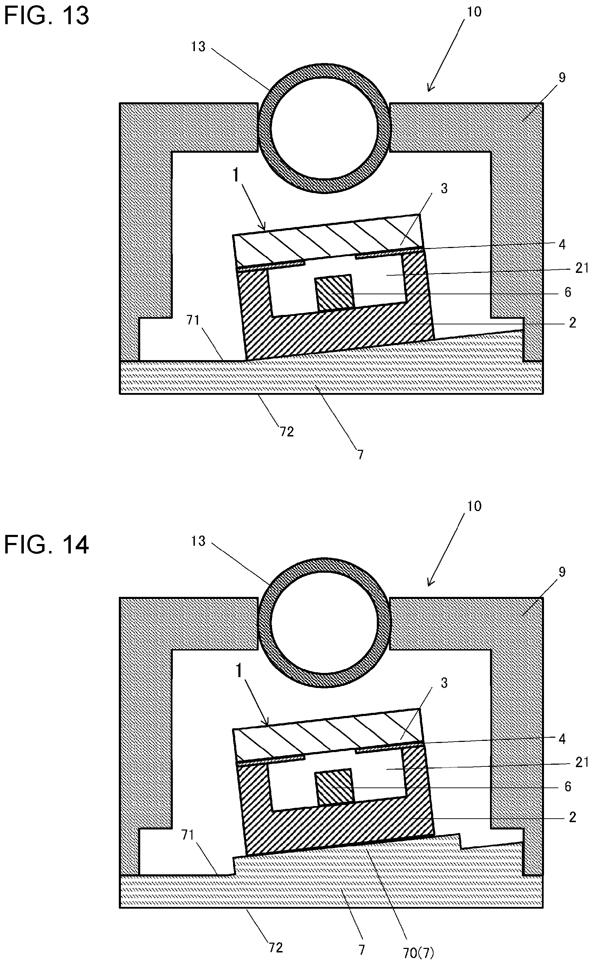

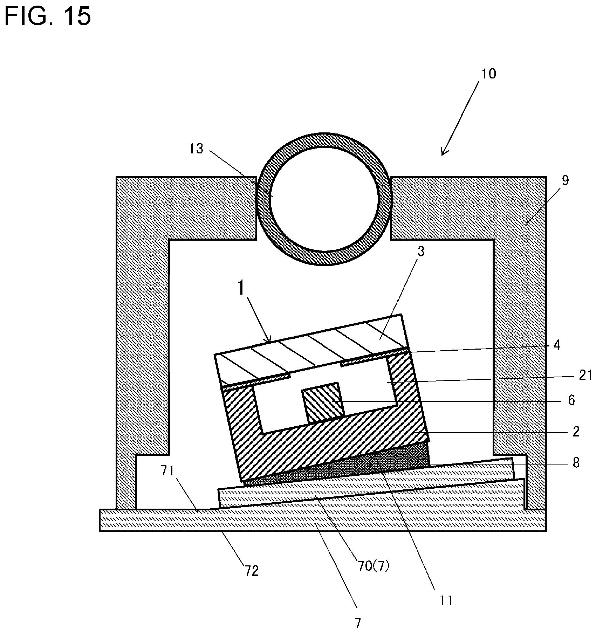

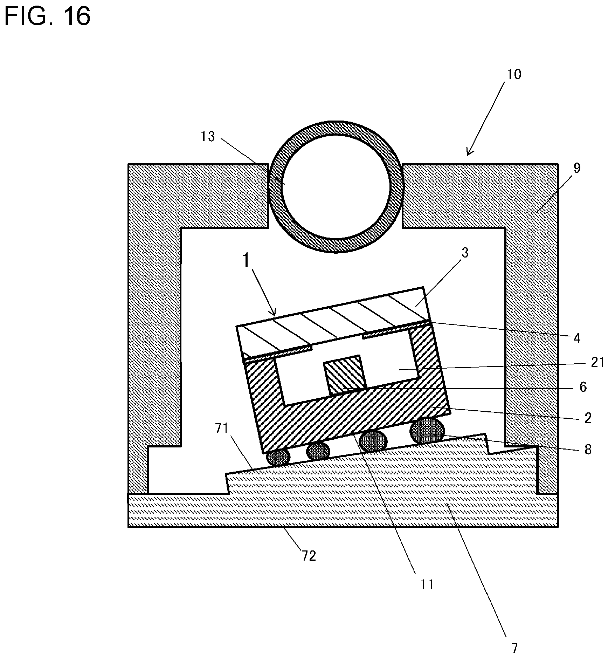

As shown in FIGs. 9 to 17, the optical sensor device 10 may further include a housing 9 and a flow passage 13, in addition to the optical sensor 1 and the mounting board 7. The housing 9 is located on the second upper surface 71 of the mounting board 7 and accommodates the optical sensor 1. As shown in FIG. 10, the mounting board 7 serves as the bottom surface of the housing 9. As shown in FIGs. 11 and 15, the mounting board 7 may further include, as a part of the mounting board 7, a mount 70 accommodated in the housing 9. As shown in FIGs. 14 to 17, the mount 70 may cause the second upper surface 71 to be stepped. The mount 70 may be formed from a different material. The optical sensor 1 has the first upper surface 23 oblique to the flow passage 13 in a transparent plan view. In this case, the optical sensor 1 may also be oblique to the second upper surface 71 of the mounting board 7. In other words, the mounting board 7 may have the second upper surface 71 inclined between the mounting board 7 and the optical sensor 1 located inside. The housing 9 may be, for example, rectangular in a plan view and may be sized differently in accordance with the dimension of the flow passage described below. The housing 9 is formed from, for example, a ceramic material or an organic material.

The flow passage 13 is attached to the housing 9. The flow passage 13 is located above the optical sensor 1 and receives fluid flowing through it. The flow passage 13 may have a circular or rectangular cross section. The flow passage 13 is sized in accordance with a fluid to be measured. The light emitter may emit light having its optical axis extending without passing through the center of the circular cross section of the flow passage 13. This allows the more specularly reflected light component to be redirected outside.

The first lower surface 11 of the optical sensor 1 may be bonded to the second upper surface 71 of the mounting board 7 parallel to each other with the bond 8 or slightly oblique to each other with the bond 8 having slightly different thicknesses. The housing 9 may have the inner bottom surface inclined more. The second upper surface 71 of the mounting board 7 and the first lower surface 11 of the optical sensor 1 may be parallel to each other with the bond 8 in between. This allows the optical sensor 1 to be oblique to the flow passage 13. This structure has a higher ratio of detectable signal light than the structure redirecting the specularly reflected light component onto the light receiver 6, and improves the detection of the intensity of the signal light.

The housing 9 has the inner bottom surface inclined in a direction perpendicular to the direction in which the fluid flows through the flow passage 13 in a cross-sectional view. In other words, the light emitter 5 emits light having its optical axis oblique to the flow passage 13. As shown in FIGs. 15 to 17, the mounting board 7 has the second upper surface 71 inclined, and the optical sensor 1 may further be mounted obliquely on the second upper surface 71. This structure has a higher ratio of detectable signal light than the structure redirecting the specularly reflected light component onto the light receiver 6, and improves the detection of the intensity of the signal light.

In the optical sensor device 10 according to the embodiment of the present disclosure, the optical sensor 1 is oblique to the second lower surface 72 of the mounting board 7 as well as to the fluid flowing through the flow passage 13 as described above. Light from the light emitter 5 thus has its optical axis oblique to the surface of the fluid or the flow passage, which serves as an object, allowing the light component specularly reflected on the surface to be redirected outside the optical sensor 1. In other words, this structure has a higher ratio of detectable signal light than the structure redirecting the specularly reflected light component onto the light receiver 6, and improves the detection of the intensity of the signal light.

### Manufacturing Method of Optical Sensor Device

A method for manufacturing the optical sensor device 10 will now be described. First, the substrate 2 is formed with a method similar to a method for manufacturing a multi-layer wiring board. For the substrate 2 that is a ceramic wiring board using alumina as a ceramic material, the powders of raw materials such as alumina (Al₂O₃), silica (SiO₂), calcium oxide (CaO), and magnesia (MgO) are mixed with an appropriate organic binder and an appropriate solvent to form slurry. The slurry is then shaped into a sheet using a known method such as a doctor blade or by calendering to obtain a ceramic green sheet (hereafter also referred to as a green sheet). The green sheet then undergoes punching into a predetermined shape. The powders of raw materials such as tungsten (W) and a glass material are mixed with an organic binder and a solvent to form a metal paste. The metal paste is then applied in a predetermined pattern by, for example, screen printing on the surface of the green sheet. The green sheet has through-holes formed and filled with the metal paste by, for example, screen printing to form via conductors. The metallized layer to be a ground conductor layer is formed on an outermost surface with the metal paste. Multiple green sheets prepared in this manner are stacked on one another, and then fired together at about 1,600 °C to complete the substrate 2.

The transparent substrate 3 is prepared by shaving or cutting a glass material into a predetermined shape. The light shield 4 (described later) is formed on the lower surface of the transparent substrate 3 by, for example, vapor deposition, sputtering, or baking.

In the above structure, the via conductors vertically extend linearly in the substrate 2. The via conductors may not extend linearly, and may be displaced inside the substrate 2 due to, for example, an inner layer interconnection or an internal ground conductor layer when the substrate 2 has the upper surface electrically connected to external connection terminals on the lower surface.

### Optical Sensor Devices According to Other Embodiments

An optical sensor device according to another embodiment of the present disclosure may include a lens attached to an upper surface of a transparent substrate 3. The lens is aligned with a first recess 21 and a first through-hole. The lens has a diameter of, for example, 20 µm to 2 mm in a plan view, and a thickness of 0.5 to 2 mm. The lens may be formed from, for example, a glass material such as quartz glass or borosilicate glass, or a resin material such as acrylic, polycarbonate, styrene, or polyolefin. The lens may be transmissive to allow light from a light emitter 5 to travel to a light receiver 6. The lens may be a collecting lens, such as a convex lens, that refracts light in the optical axis direction. The lens refracts diffusive light from the light emitter 5 into convergent or collimated light, and thus can more effectively collect light to be directed to the light receiver 6.

The optical sensor device may further include a second lens attached to the upper surface of the transparent substrate 3. The second lens is aligned with a second recess 22 and a second through-hole. The second lens has a diameter of, for example, 70 µm to 2 mm in a plan view, and a thickness of 50 µm to 2 mm. The second lens may be formed from, for example, a glass material such as quartz glass or borosilicate glass, or a resin material such as acrylic, polycarbonate, styrene, or polyolefin. The second lens may be transmissive to allow light from the light emitter 5 to pass through. The second lens may be a collecting lens, such as a convex lens, that refracts light in the optical axis direction. The second lens refracts diffusive light from the light emitter 5 into convergent or collimated light, and thus can more effectively collect light.

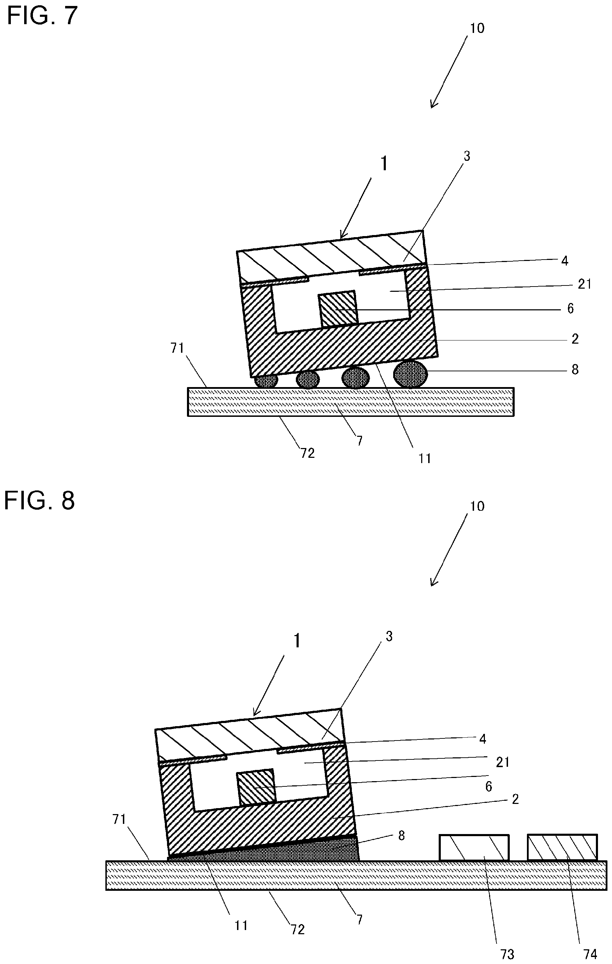

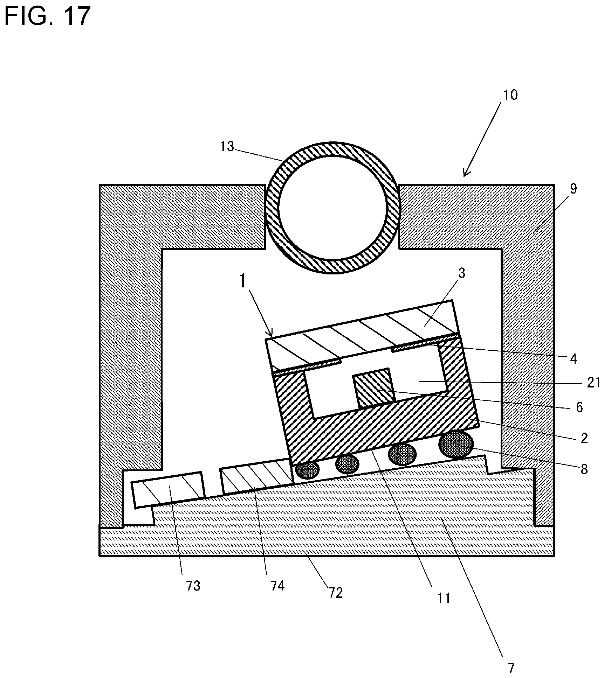

As shown in FIGs. 8 and 17, for example, a controller for controlling light emission from the light emitter 5, a processor 73 for processing output signals from the light receiver 6, and an arithmetic unit 74 for calculating the blood flow rate and other parameters based on signals from the processor 73 are also mounted on a mounting board 7.

To start measurement, the fingertip of a finger to be a measurement object is placed into contact with the surface of the transparent substrate 3. In this state, a light emitter control current is provided from the mounting board 7 into the optical sensor device 10 through an external connection terminal, and input into the light emitter 5 through a via conductor and a connection pad. Light for measurement is then emitted from the light emitter 5. When the emitted light is applied to the fingertip through the transparent substrate 3, the light is scattered by blood cells in the blood. When receiving the scattered light transmitted through the transparent substrate 3, the light receiver 6 outputs an electric signal corresponding to the amount of received light. The output signal then passes through the connection pad and the via conductor, and is output from an optical sensor 1 to the processor 73 on the mounting board 7 through the external connection terminal.

On the mounting board 7, a signal processed by the processor 73 is input into the arithmetic unit 74 including an arithmetic element, which can then calculate the blood flow velocity by analyzing the intensity of scattered light received by the light receiver 6 for each frequency.

An optical sensor device 10 according to another embodiment of the present disclosure may further include a second light receiver accommodated in a first recess 21. In this embodiment, a light shield 4 may have an opening over the second light receiver to receive light illuminated toward the second light receiver. This reduces ambient light reaching the second light receiver.

In an optical sensor device 10 according to another embodiment of the present disclosure, a light shield 4 may further have a through-hole that aligns with a second recess. The through-hole receives reference light. The through-hole in the light shield is located closer to a light receiver, allowing the light receiver to receive reference light. This allows light to travel to a light receiver 6 more precisely, thus enabling more accurate velocity calculation.

In an optical sensor device 10 according to another embodiment of the present disclosure, a clearance is left between a lower surface of a transparent substrate 3 and a substrate 2 between a first recess 21 and a second recess 22. More specifically, the substrate 2 includes a light-shield wall between the first recess 21 and the second recess 22. The light-shield wall has a partially recessed upper end that allows reference light to pass and directly reach a light receiver 6, thus allowing more accurate measurement.

In an optical sensor device 10 according to another embodiment of the present disclosure, an optical sensor 1 may be rectangular having long sides and short sides in a plan view, and the long sides may be parallel to a first imaginary straight line X1. In this case, the optical sensor 1 may have its short sides parallel to a second imaginary straight line X2. This allows the optical sensor 1 to be easily mounted obliquely on the mounting board 7.

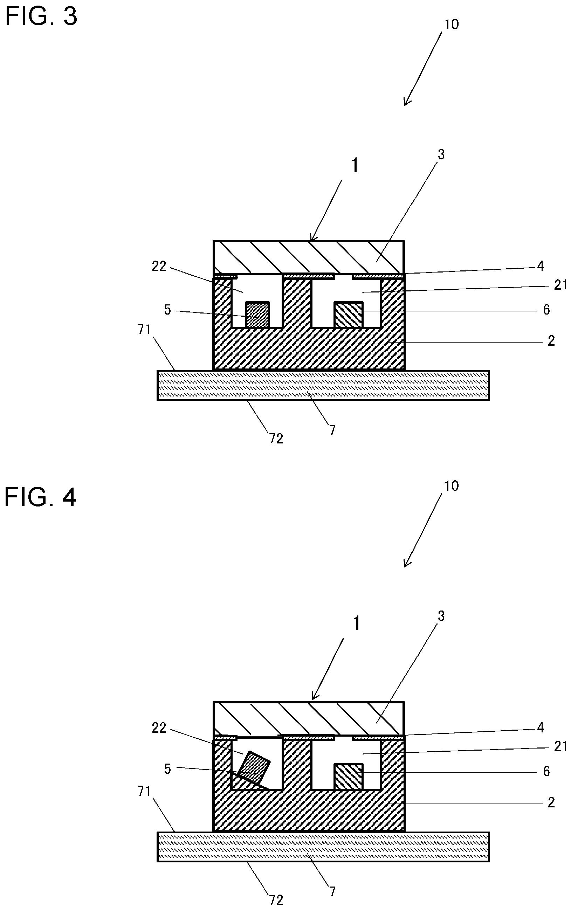

As shown in FIGs. 3 and 12, an optical sensor device 10 according to another embodiment of the present disclosure may have a first recess 21 and a second recess 22 each having an inclined bottom surface. Each inclined bottom surface may have an inclination angle of, for example, 5 to 50° with respect to a first upper surface 23. This inclination allows a measurement object to receive light from a nearer illumination position.

The present disclosure is not limited to the examples described in the above embodiments and may include various modifications of, for example, numerical values. Any method may be used for mounting the units or devices in the embodiments. All the features of one or more of the embodiments according to the present disclosure may be combined unless any contradiction arises. Although the optical sensor devices according to the embodiments of the present disclosure descried above are used as pulse wave blood flow sensor devices, the devices may be used in other devices operable with a pair of sensor elements including a light emitter and a light receiver, such as proximity illuminance sensor devices, proximity sensor devices, and distance measuring sensor devices.

### Reference Signs List

- 1: optical sensor
- 11: first lower surface
- 2: substrate
- 21: first recess
- 22: second recess
- 23: first upper surface
- 3: transparent substrate
- 4: light shield
- 5: light emitter
- 6: light receiver
- 7: mounting board
- 70: mount
- 71: second upper surface
- 72: second lower surface
- 73: processor
- 74: arithmetic unit
- 8: bond
- 9: housing
- 21: first recess
- 22: second recess
- 10: optical sensor device
- X1: first imaginary straight line
- X2: second imaginary straight line

## Claims

1. An optical sensor device, comprising:
an optical sensor having a first upper surface and a first lower surface; and
a mounting board having a second upper surface and a second lower surface, the mounting board receiving the optical sensor,
wherein the optical sensor includes
a substrate having a first recess and a second recess at a distance from the first recess,
a light receiver in the first recess,
a light emitter in the second recess, and
a transparent substrate on the substrate, the transparent substrate is bonded to the substrate and covers the first recess and the second recess, and
the first lower surface is oblique to the second lower surface.

2. The optical sensor device according to claim 1, wherein
the first lower surface is oblique to the second lower surface in a cross-sectional view taken along a second imaginary straight line perpendicular to a first imaginary straight line connecting a center of the light receiver and a center of the light emitter in a transparent plan view.

3. The optical sensor device according to claim 2, wherein
the optical sensor is rectangular and has long sides and short sides in a plan view, and the long sides are parallel to the first imaginary straight line.

4. The optical sensor device according to any one of claims 1 to 3, further comprising:
a housing on the second upper surface of the mounting board, the housing accommodating the optical sensor; and
a flow passage attached to the housing and located above the optical sensor, the flow passage being configured to receive fluid flowing through the flow passage,
wherein the optical sensor has the first upper surface oblique to the flow passage in a transparent plan view.

5. The optical sensor device according to any one of claims 1 to 4, wherein
the first lower surface is parallel to the second upper surface.

6. The optical sensor device according to claim 4 or claim 5, wherein
the light emitter emits light having an optical axis oblique to the flow passage.

7. The optical sensor device according to any one of claims 4 to 6, wherein
the flow passage has a circular cross section as viewed in a direction perpendicular to a direction in which the fluid flows through the flow passage, and
the light emitter emits light having an optical axis extending without passing through a center of the circular cross section of the flow passage.

8. The optical sensor device according to any one of claims 1 to 7, wherein
the second recess has an inclined bottom surface inclined upward toward the first recess.

9. The optical sensor device according to any one of claims 1 to 8, wherein
a bond is between the optical sensor and the mounting board.

10. The optical sensor device according to claim 9, wherein
the bond is across the first lower surface.

11. The optical sensor device according to claim 9 or claim 10, wherein
the bond includes a plurality of solder balls, and
the plurality of solder balls include a first solder ball at a first position on the first lower surface at which the optical sensor is at a first distance from the mounting board and a second solder ball at a second position on the first lower surface at which the optical sensor is at a second distance greater than the first distance from the mounting board, and the second solder ball has a larger diameter than the first solder ball.

12. The optical sensor device according to claim 9 or claim 10, wherein
the plurality of solder balls include a solder ball with a larger diameter at a position on the first lower surface at which the optical sensor is at a greater distance from the mounting board.

13. The optical sensor device according to any one of claims 1 to 12, wherein
the mounting board includes a processor configured to process light received by the light receiver.

14. The optical sensor device according to claim 13, wherein
the mounting board includes an arithmetic unit configured to perform an arithmetic operation based on a signal processed by the processor.
